# EUROPÄISCHE PATENTSCHRIFT

(11) **EP 3 469 500 B1**
(45) Veröffentlichungstag und Bekanntmachung des Hinweises auf die Patenterteilung: **28.02.2024**
(21) Anmeldenummer: 17730385.6
(22) Anmeldetag: 22.05.2017
(51) Int. Cl.: G16H 20/10

(54) **VERFAHREN UND SYSTEM ZUR UNTERSTÜTZUNG DER PLANUNG EINER MEDIKATION FÜR EINEN PATIENTEN**
METHOD AND SYSTEM FOR ASSISTING IN THE PLANNING OF MEDICATION FOR A PATIENT
PROCÉDÉ ET SYSTÈME D'ASSISTANCE À LA PRESCRIPTION D'UNE MÉDICATION POUR UN PATIENT

(30) Priorität: 08.06.2016 DE 102016210158
(43) Veröffentlichungstag der Anmeldung: 17.04.2019
(73) Patentinhaber: HMG Systems Engineering GmbH, 90763 Fürth (DE)
(72) Erfinder: MUNOZ-GALEANO, Herna, 90763 Fürth (DE); HERFORTH, Matthias, 24226 Heikendorf (DE); PEDRAZA, Angela, 90408 Nürnberg (DE); KIDANE, Ariam, 90461 Nürnberg (DE); HE, Wei, 90763 Fürth (DE); ZAMBRANO, Paola, 90766 Fürth (DE); MEDINA-WARMBURG, Cristian, 90763 Fürth (DE); HUSSNÄTTER, Michael, 90768 Fürth (DE)
(74) Vertreter: FDST Patentanwälte
(86) Internationale Anmeldenummer: PCT/EP2017/062293
(87) Internationale Veröffentlichungsnummer: WO 2017/211581

(56) Entgegenhaltungen:
- US-A1- 2009 094 059
- US-A1- 2011 082 867

## Beschreibung

Die Erfindung bezieht sich auf ein Verfahren zur Unterstützung der Planung einer Medikation für einen Patienten. Die Erfindung bezieht sich weiterhin auf ein zugehöriges System. Der Begriff "System" bezeichnet hierbei eine Kombination einer Software (d.h. eines Computerprogramms) zur Durchführung des Verfahrens, und einer Hardware-Einrichtung, auf der die Software lauffähig implementiert ist.

Das Verfahren und das zugehörige System dienen insbesondere zur Unterstützung von Medizinern (Ärzten) bei der medizinischen Ursachenforschung und Linderung der Probleme insbesondere "multimorbider", und daher häufig "polymedikamentierter" Patienten sowie bei der Findung einer alternativen, und wünschenswerterweise verbesserten Medikation für solche Patienten.

Als "multimorbide" werden Patienten bezeichnet, bei denen komplexe und vielschichtige Krankheitsbilder auftreten. Ein typischer Fall eines solchen komplexen Krankheitsbildes ist beispielsweise durch das gleichzeitige Auftreten von Kreislaufstörungen, Diabetes und Depressionen gekennzeichnet.

Als "polymedikamentiert" werden Patienten bezeichnet, die - insbesondere als Folge komplexer Krankheitsbilder - eine Vielzahl von Medikamenten parallel zueinander einnehmen müssen. Derzeit wird von Polymedikation gesprochen, wenn mindestens fünf verschiedene Medikamente zeitlich parallel eingenommen werden.

Der Erfolg oder Misserfolg einer Medikation multimorbider Patienten (sowie der Erfolg oder Misserfolg einer Polymedikation im Allgemeinen) ist auch für erfahrene Ärzte vor der Anwendung kaum abzuschätzen, da hier eine Vielzahl von Einflussfaktoren miteinander wechselwirken können.

Das entsprechend hohe Risiko für Fehlmedikationen oder erfolglose Medikationen resultiert in einem hohen Leidensdruck sowie mitunter gesundheitlichen Folgeschäden für die betroffenen Patienten sowie in erheblichen Kosten für das Gesundheitswesen.

Ein Verfahren nach dem Oberbegriff des Anspruchs 1 und ein System nach dem Oberbegriff des Anspruchs 6 sind aus US 2011/0082867 A1 bekannt.

Der Erfindung liegt die Aufgabe zugrunde, die Planung einer Medikation zu vereinfachen.

Als "Medikation" eines Patienten wird hier und im Folgenden die Gesamtheit der von dem Patienten zeitlich parallel einzunehmenden Medikamente, einschließlich der Eigenmedikation des Patienten, mit zugehörigen Angaben zur Dosierung und Verabreichung (Zeitpunkt und Darreichungsform) dieser Medikamente bezeichnet. Es wird dabei im Regelfall von einer Medikation ausgegangen, die mehrere (insbesondere mindestens fünf) Medikamente umfasst. In bestimmten Einzelfällen kann die Medikation im Rahmen der Erfindung aber auch nur ein einzelnes Medikament umfassen.

Die "Planung" einer Medikation umfasst einerseits die Überprüfung der aktuell angewendeten Medikation des Patienten, sowie andererseits die Findung einer verbesserten Medikation für die zukünftige Anwendung. Die Planung umfasst dabei insbesondere die Erkennung von Risiken (d.h. kontraproduktiven Einflüssen auf die Gesundung des Patienten), die durch die Anwendung einer gegebenen Medikation eingetreten oder zu erwarten sind, und die Erkennung der Ursachen solcher Risiken.

Bezüglich eines Verfahrens wird die obige Aufgabe erfindungsgemäß gelöst durch die Merkmale des Anspruchs 1. Bezüglich eines Systems wird Aufgabe erfindungsgemäß gelöst durch die Merkmale des Anspruchs 6. Vorteilhafte und teils für sich gesehen erfinderische Ausgestaltungen und Weiterbildungen der Erfindung sind in den Unteransprüchen und der nachfolgenden Beschreibung dargelegt.

Das erfindungsgemäße Verfahren zur Unterstützung der Medikationsplanung umfasst folgende Schritte:
a) Es wird eine Wirkstoff- und Interaktionsdatenbank bereitgestellt, die
   - Informationen zu Wirkstoffen und Anwendungsangaben von Medikamenten,
   - Informationen zu Wirkstoffen von Lebensmitteln, umfassend Nahrungsmittel, Nahrungsergänzungsmittel, Genussmittel, Kräuter und/oder Betäubungsmittel, und
   - Informationen zu Auswirkungen von Lebensumständen, umfassend Alter, Geschlecht, Körpergewicht, Schwangerschaft, Organfunktionen und/oder Strahlungsbelastung, auf die Wirkung von Medikamenten bzw. Lebensmitteln,
   umfasst.
b) Es wird eine pharmakogenetische Datenbank bereitgestellt, die pharmakogenetische Information, nämlich Informationen zu genetischen Einflüssen auf die Metabolisierung (d.h. "Verstoffwechselung") und/oder den Transport bzw. die Rezeptorbindung von Wirkstoffen aus Medikamenten und/oder Lebensmitteln enthält.
c) Es werden Patientendaten eines Patienten erfasst, die Angaben zur Einnahme bestimmter Lebensmittel und/oder Angaben zu (sonstigen) Lebensumständen umfassen.
d) Es werden Angaben zum Krankheitsbild des Patienten erfasst, die Angaben zu diagnostizierten Krankheiten und/oder Beschwerden umfassen; des Weiteren wird eine aktuelle Medikation des Patienten erfasst.
e) Es werden unter Zugriff auf die Wirkstoff- und Interaktionsdatenbank Risiken für eine abnormal verstärkte oder abgeschwächte Wirkung von Wirkstoffen der aktuellen Medikation und/oder der eingenommenen Lebensmittel ermittelt, die mit den Patientendaten korreliert sind.
f) Es wird eine Genanalyse des Patienten erfasst.
g) Es werden bestimmte genotypologische Merkmale aus der Genanalyse extrahiert.
h) Es werden unter Zugriff auf die pharmakogenetische Datenbank pharmakogenetische Risiken für eine abnormal verstärkte oder abgeschwächte Wirkung von Wirkstoffen der aktuellen Medikation und/oder der eingenommenen Lebensmitteln ermittelt, die mit den genotypologischen Merkmalen korreliert sind.
i) Es wird automatisch ein Bericht erstellt anhand
   - der in Schritt e) ermittelten, mit den Patientendaten korrelierten Risiken, und
   - der in Schritt h) ermittelten pharmakogenetischen Risiken.

Die vorstehend aufgeführten Verfahrensschritte können, soweit dies aus Kausalitätsgründen möglich ist, im Rahmen der Erfindung in beliebiger zeitlicher Abfolge durchgeführt werden. Insbesondere können dabei auch mehrere der oben genannten Schritte gleichzeitig, also zeitlich parallel oder zeitlich überlappend durchgeführt werden.

Der Begriff "Lebensmittel" wird hier und im Folgenden allgemein als Oberbegriff für alle Produkte, Stoffe und Substanzen verwendet, die bewusst durch Verzehr, Inhalation, äußerliche Auftragung oder auf sonstige Weise zur Aufnahme in dem menschlichen Organismus zugeführt werden, die aber nicht zu den Medikamenten zählen. Der Begriff "Lebensmittel" umfasst somit im Kontext dieser Anmeldung neben gewöhnlichen Nahrungsmitteln auch Genussmittel, Kräuter und/oder Betäubungsmittel, insbesondere Nahrungsergänzungsmittel, Tabakwaren, Alkohol, Drogen und Kosmetika.

Der Begriff "Risiko" bezeichnet hier und im Folgenden allgemein eine erhöhte Wahrscheinlichkeit dafür, dass ein bestimmter Wirkstoff bei dem Patienten eine abnormal (d.h. außergewöhnlich oder deutlich vom Normalfall abweichend) verstärkte oder abgeschwächte Wirkung hervorruft und sich somit potentiell kontraproduktiv auf die Gesundung des Patienten auswirkt. Die kontraproduktive Folge einer abgeschwächten Wirkung eines Wirkstoffs äußert sich dabei insbesondere in der Wirkungslosigkeit der verordneten Medikation oder in einem verstärkten Auftreten von Nebenwirkungen anderer Wirkstoffe der Medikation. Eine gesteigerte Wirkung eines Wirkstoffs führt dagegen häufig zu einer Überdosierung und, damit verbunden, wiederum zu einem verstärkten Auftreten von Nebenwirkungen.

Die erfassten Patientendaten enthaltenen vorzugsweise Angaben zum Konsum von bestimmten Nahrungsmitteln (z.B. Häufigkeit des Gewürz-, des Kräuter- und/oder des Obst- und Gemüsekonsums) und/oder Nahrungsergänzungsmitteln (z.B. Häufigkeit des Vitaminkonsums). Zusätzlich oder alternativ werden in den Patientendaten als Lebensgewohnheiten der Konsum von Genuss- oder Betäubungsmitteln (z.B. Tabak, Koffein, Alkohol und/oder Drogen) erfasst. Weiterhin umfassen die Patientendaten vorzugsweise Angaben zu dem Alter, dem Geschlecht, der Größe und/oder dem Körpergewicht des Patienten sowie ggf. weiteren Lebensumständen (z.B. Schwangerschaft, außergewöhnliche Strahlungsbelastung, Ausmaß der sportlichen Betätigung/Bewegung, Stress, etc.).

Zusätzlich zu den vorstehend genannten Angaben werden vorzugsweise auch Angaben zur Funktion von Organen des Patienten, insbesondere zur Nierenfunktion und/oder Leberfunktion erfasst.

Die in der Wirkstoff- und Interaktionsdatenbank enthaltenen Anwendungsangaben zu Medikamenten enthalten vorzugsweise Angaben zu der Dosierung, den gewöhnlichen Verabreichungsformen sowie den gewöhnlichen Verabreichungszeiten. Darüber hinaus enthält die Wirkstoff- und Interaktionsdatenbank vorzugsweise auch Angaben zu den Anwendungsgebieten (Indikationen), therapeutischen Wirkungen, Nebenwirkungen und Gegenanzeigen (Kontraindikationen) von Medikamenten sowie Angaben zu deren Wirkstoffzusammensetzung. Die Wirkstoff- und Interaktionsdatenbank bezieht diese Information insbesondere aus öffentlich zugänglichen Quellen, z.B. Beipackzetteln oder einer Apothekerdatenbank.

In einer bevorzugten Variante des Verfahrens enthält die Wirkstoff- und Interaktionsdatenbank zusätzlich Informationen zu Wechselwirkungen zwischen Medikamenten und/oder Lebensmitteln. Alternativ hierzu enthält die Wirkstoff- und Interaktionsdatenbank Informationen zu Wechselwirkungen zwischen den in Medikamenten bzw. Lebensmitteln enthaltenen Wirkstoffen. In diesen Verfahrensvarianten werden im Zuge des Verfahrens Wechselwirkungen der in der aktuellen Medikation des Patienten enthaltenen Medikamente und/oder der von dem Patienten eingenommenen Lebensmittel oder die Wechselwirkungen der jeweiligen Wirkstoffe aus der Wirkstoff- und Interaktionsdatenbank ermittelt. Diese ermittelten Wechselwirkungen werden bei der Erstellung des Berichts berücksichtigt. Insbesondere werden die ermittelten Wechselwirkungen in dem Bericht als zusätzliche Information aufgeführt.

Die aus der Genanalyse abgeleiteten genotypologischen Merkmale enthalten vorzugsweise Angaben zu Einzelnukleotid-Polymorphismen (Single-Nucleotide Polymorphisms, kurz SNPs), Indels und/oder Kopienzahlvarianten (Copy Number Variations, kurz CNVs), die in dem Genotyp des Patienten enthalten sind.

Erfindungsgemäß wird anhand der genotypologischen Merkmale des Patienten, der aktuellen Medikation und der Wirkstoff- und Interaktionsdatenbank automatisch eine alternative Medikation ermittelt und in dem Bericht angegeben. Die Angabe der alternativen Medikation in dem Bericht kann dabei aber zweckmäßigerweise in Einzelfällen auch unterbleiben, wenn keine adäquate alternative Medikation gefunden wird (z.B. weil es zu bestimmten Medikamenten aus der aktuellen Medikation keine Alternativen gibt, oder weil gefundene Alternativen mit zu hohen Neben- oder Wechselwirkungen oder anderweitigen Risiken verbunden sind).

In zweckmäßiger Ausgestaltung des Verfahrens unterscheidet sich die alternative Medikation von der aktuellen Medikation in mindestens einem Medikament. Es ist also mindestens ein Medikament der aktuellen Medikation ersatzlos weggelassen oder durch mindestens ein anderes Medikament ausgetauscht.

Zur Ermittlung der alternativen Medikation werden beispielsweise aus der Wirkstoff- und Interaktionsdatenbank die therapeutischen Wirkungen der Medikamente der aktuellen Medikation ermittelt. Anhand dieser Wirkungen wird aus der Wirkstoff- und Interaktionsdatenbank eine andere Kombination von Medikamenten gesucht, die hinsichtlich der Gesamtheit ihrer therapeutischen Wirkungen der aktuellen Medikation entspricht.

Vorzugsweise werden auch für die in der alternativen Medikation enthaltenen Medikamente oder deren Wirkstoffe etwaige Neben- und Wechselwirkungen aus der Wirkstoff- und Interaktionsdatenbank ermittelt. Des Weiteren werden aus der pharmakogenetischen Datenbank die mit der alternativen Medikation verbundenen pharmakogenetischen Risiken ermittelt. Auch hier bezeichnet der Begriff "pharmakogenetisches Risiko" eine erhöhte Wahrscheinlichkeit für eine abnormal verstärkte oder abgeschwächte Wirkung eines bestimmten Wirkstoffs, die mit bestimmten genotypologischen Merkmalen des Patienten korreliert ist. In dem Bericht wird hierbei die alternative Medikation zusammen mit den zugehörigen Neben- und Wechselwirkungen und den zugehörigen pharmakogenetischen Risiken angegeben.

In einer Variante der Erfindung werden mehrere alternative Medikationen ermittelt und, insbesondere jeweils mit den zugehörigen Neben- und Wechselwirkungen und den zugehörigen pharmakogenetischen Risiken, in dem Bericht angeführt. Die alternativen Medikationen sind dabei vorzugsweise in einer Reihenfolge in dem Bericht aufgeführt, die durch den Grad der zugehörigen pharmakogenetischen Risiken und den Grad der zugeordneten Neben- und Wechselwirkungen bestimmt ist. Alternative Medikationen, die mit fehlenden oder nur leichten pharmakogenetischen Risiken und fehlenden oder nur leichten Neben- und Wechselwirkungen verbunden sind, sind dabei in dem Bericht vor anderen alternativen Medikationen aufgeführt, die mit stärkeren pharmakogenetischen Risiken und/oder stärkeren Neben- und Wechselwirkungen verbunden sind.

Erfindungsgemäß ist weiterhin vorgesehen, dass Erfahrungen des Patienten und/oder des behandelnden Arztes mit der verfahrensgemäß ermittelten alternativen Medikation wieder in das Verfahren und das System zurückgespeist werden, um das Verfahren bzw. das System zu verbessern. Hierbei ist vorgesehen, dass nach einer Anwendung der alternativen Medikation auf den Patienten eine Rückmeldung zu dem Ergebnis der alternativen Medikation (insbesondere einer Verbesserung oder Verschlechterung der Krankheitssymptome oder Beschwerden des Patienten) erfasst wird. Anhand dieser Rückmeldung wird die pharmakogenetische Datenbank dann aktualisiert, und somit insbesondere in ihrer Aussagekraft verbessert.

In einer beispielhaften Ausführung erfolgt die Aktualisierung der pharmakogenetischen Datenbank dadurch, dass derjenigen pharmakogenetischen Information (insbesondere diejenigen Zusammenhänge zwischen genotypologischen Merkmalen und der Metabolisierung bestimmter Wirkstoffe), auf der die Ermittlung der zugrundeliegenden alternativen Medikation beruhte, je nach positiver oder negativer Rückmeldung in der pharmakogenetischen Datenbank eine entsprechende Bewertung zugeordnet wird. Pharmakogenetische Informationen mit überwiegend positiven Bewertungen werden dabei beispielsweise durch die pharmakogenetische Datenbank mit höherer Priorität angeboten, während pharmakogenetische Informationen mit überwiegend negativen Bewertungen mit geringer Priorität angeboten werden.

Um kontraproduktive Auswirkungen von falschen oder unvollständigen Rückmeldungen auf die pharmakogenetische Datenbank zu vermeiden, wird jede Rückmeldung vorzugweise nach vorgegebenen Kriterien auf Qualität geprüft, wobei die Rückmeldung nur dann für die Aktualisierung der pharmakogenetischen Datenbank freigegeben wird, wenn die Rückmeldung nach Maßgabe der Prüfung eine vorgegebene Mindestqualität erreicht oder übersteigt. Als Kriterien der Qualitätsprüfung werden insbesondere die Vollständigkeit und Plausibilität der in der Rückmeldung enthaltenen Angaben herangezogen. Die Qualitätsprüfung erfolgt vorzugsweise vollautomatisch durch das System. Im Rahmen der Erfindung kann alternativ aber vorgesehen sein, dass die Qualitätsprüfung durch einen medizinischen oder pharmakogenetischen Experten in Interaktion mit dem System durchgeführt oder zumindest überwacht wird.

In einer weiteren Ausführungsform der Erfindung wird die pharmakogenetische Datenbank, insbesondere fortlaufend (d.h. zeitlich kontinuierlich) mit öffentlich zugänglichen wissenschaftlichen Erkenntnissen auf dem Gebiet der Pharmakogenetik aktualisiert. Vorzugsweise werden hierbei mittels einer speziell für diesen Zweck implementierten Suchmaschine im Internet wissenschaftliche Publikationen auf dem Gebiet der Pharmakogenetik recherchiert, deren Inhalt nach einem festgelegten Verfahren extrahiert und in die pharmakogenetische Datenbank eingepflegt wird.

Um eine kontraproduktive Beeinflussung der pharmakogenetischen Datenbank durch fragwürdige Publikationen zu unterbinden, ist dieser Aktualisierung der pharmakogenetischen Datenbank vorzugsweise ein Validierungsprozess vorgeschaltet. Dabei wird in einer vorteilhaften Ausführung dieses Prozesses der Evidenzgrad (Level of Evidence) einer jeden recherchierten Publikation bewertet. Der Inhalt der jeweiligen Publikation wird dabei nur dann zur Aktualisierung der pharmakogenetischen Datenbank verwendet, wenn die Bewertung eine vorgegebene Zulassungsschwelle übersteigt. Die Bewertung erfolgt vorzugsweise automatisiert, insbesondere unter Nutzung statistischer Kriterien. Als Bewertungskriterien werden insbesondere
- die Art der Studien (z.B. Meta-Analyse, randomisierte kontrollierte Studie, kontrollierte Studie ohne Randomisierung, Kohortenstudie, Fall-Kohortenstudie, Querschnittsstudie)
- das Vorliegen oder Fehlen von Empfehlungen anerkannter Authoritäten, z.B. der US Food and Drug Administration (FDA), und der
- Qualität und Konsistenz der einzelnen Studien.

In zweckmäßiger Ausführung der Erfindung wird z.B. ein dreistufiges Klassifizierungsschema angewandt, bei dem der Evidenzgrad einer Publikation
- als hoch eingestuft wird, wenn sie auf methodisch hochwertigen Studien beruht; Anzeichen hierfür sind die Bezugnahme auf Meta-Analysen oder randomisierte kontrollierte Studien, oder eine FDA-Empfehlung,
- als mittel eingestuft wird, wenn die untersuchten Effekte aufgrund der Anzahl, Qualität oder der Konsistenz der Einzelstudien, aufgrund fehlender Verallgemeinerungsfähigkeit auf die routinemäßige Praxis, oder indirekter Beweisführung nur mit mäßiger Aussagekraft nachgewiesen sind; Anzeichen hierfür sind die ausschließliche Bezugnahme auf Beobachtungsstudien mit retrospektiver Kontrolle oder ohne Kontrolle (z.B. Kohorten-Studien oder Fallserien)
- als gering eingestuft wird, wenn aufgrund geringer Anzahl oder Qualität der Studien, methodischer Fehlern, Lücken in der Beweiskette oder fehlender Information ein gesundheitlicher Effekt nicht mit ausreichender Aussagekraft nachgewiesen ist; Anzeichen hierfür sind u.a., wenn die Studie ausschließlich auf Expertenmeinungen oder deskriptive Studien Bezug nimmt.

In einer verfeinerten Ausgestaltung der Erfindung wird jede recherchierte Publikation in einem mehrstufigen Validierungsprozess auf inhaltliche Relevanz, Evidenzgrad, die Beschreibung mindestens eines pharmakokinetischen Effekts und/oder klinischen Konsequenz bezüglich einer genetischen Variation sowie auf inhaltliche Übereinstimmung (Redundanz) mit mindestens einer weiteren Publikation geprüft. Der Inhalt der Publikation wird dabei nur dann zur Aktualisierung der pharmakogenetische Datenbank herangezogen, wenn die Publikation die Anforderungen aller Stufen des Validierungsprozesses erfüllt.

Die erste Stufe des mehrstufigen Validierungsprozesses bildet vorzugsweise eine Vorsortierung ("Screening") der durch die Suchmaschine gefundenen Publikationen PB hinsichtlich ihrer jeweiligen thematischen Relevanz. Die Vorsortierung beruht in zweckmäßiger Ausführung auf einem Vergleich der zu testenden Publikationen PB mit vorgegebenen (und nutzerseitig veränderbaren) Textblöcken, d.h. komplexen Suchbegriffen aus mehreren Suchwörtern. Die Publikationen werden dabei vorzugsweise nach Maßgabe der festgestellten thematischen Relevanz in verschiedene thematische Listen eingegliedert.

In einer zweiten Stufe des mehrstufigen Validierungsprozesses erfolgt vorzugsweise die Bewertung der als thematisch relevant befundenen Publikationen hinsichtlich ihres Evidenzgrades. Zusätzlich werden die evaluierten Publikationen vorzugsweise daraufhin überprüft, ob sie einen oder mehrere pharmakokinetische Effekte und/oder klinische Konsequenzen bezüglich einer nachgewiesenen genetischen Variation beschreiben.

Publikationen mit hinreichendem Evidenzgrad, die diese zusätzliche Anforderung erfüllen, werden vorzugsweise in einer dritten Stufe des mehrstufigen Validierungsprozesses einem Redundanztest unterzogen. Dabei werden die Publikationen, die die zweite Stufe erfolgreich durchlaufen haben, vergleichend auf inhaltliche Übereinstimmung ausgewertet. Sofern eine bestimmte Anzahl von Publikationen (insbesondere mindestens zwei voneinander unabhängige Publikationen) einen bestimmten pharmakogenetischen Zusammenhang übereinstimmend darstellen, wird der Inhalt dieser Publikationen (insbesondere dieser pharmakogenetischen Zusammenhang) zur Aufnahme in die pharmakogenetische Datenbank freigegeben.

Die pharmakogenetische Information der solchermaßen validierten Publikationen wird vorzugsweise automatisch extrahiert und in die Datenbank eingepflegt.

Der vorstehend beschriebene Validierungsprozess kann im Rahmen der Erfindung vollautomatisch (ohne menschlichen Eingriff) durchgeführt werden. Vorzugsweise wird der Validierungsprozess aber von einem pharmakogenetischen Experten oder einem Expertengremium gesteuert und/oder überwacht.

Das erfindungsgemäße System ist durch eine auf einer geeigneten Hardware-Struktur lauffähig implementierte Software gebildet. Die Hardware-Struktur umfasst insbesondere einen mit dem Internet datenübertragungstechnisch verbundenen Rechner oder ein aus mehreren Rechnern und ggf. separaten Speichereinheiten gebildetes Rechnernetzwerk. Das System ist durch entsprechende Programmierung der Software allgemein zur Durchführung des vorstehend beschriebenen erfindungsgemäßen Verfahrens eingerichtet, so dass beim bestimmungsgemäßen Ablauf der Software das Verfahren durchgeführt wird. Das System weist entsprechend eine Anzahl von Modulen auf, die die vorstehend beschriebenen Schritte des erfindungsgemäßen Verfahrens funktional implementieren.

Als "Modul" wird dabei ein aus Software und/oder Hardware gebildeter Systembestandteil mit einer bestimmten Funktion bezeichnet. Ein solches Modul ist z.B. als Unterprogramm oder als Objekt oder Komponente (im Sinne einer objekt- bzw. komponentenorientierten Programmierung) ausgestaltet. Im Rahmen der Erfindung können aber mehrere der genannten Module zu einer größeren Software- und/oder Hardware-Einheit zusammengefasst sein. Ebenso kann im Rahmen der Erfindung ein Modul aus mehreren separaten (Unter-)Modulen zusammengesetzt sein. Wenn im Folgenden eine bestimmte Funktion "einer Anzahl von Modulen" zugeordnet ist, so wird damit ausgedrückt, dass diese Funktion vorzugsweise auf mehrere Module verteilt ist, im Rahmen der Erfindung grundsätzlich aber auch in einem einzigen Modul implementiert sein kann. Der Begriff "eine Anzahl von Modulen" wird somit synonym mit "ein oder mehrere Module" verwendet.

Die vorstehend beschriebenen Ausführungsformen und Weiterentwicklungen des erfindungsgemäßen Verfahrens finden ihre Entsprechung in zugehörigen Varianten des Systems. In Hinblick auf bevorzugte Ausführungsformen und Weiterentwicklungen des erfindungsgemäßen Systems wird daher entsprechend auf die vorstehenden Ausführungen zu dem Verfahren Bezug genommen.

Die zwei Datenbanken, auf die im Zuge des Verfahrens zugegriffen wird, nämlich
- die Wirkstoff- und Interaktionsdatenbank, und
- die pharmakogenetische Datenbank,
bilden vorzugsweise integrale Bestandteile des Systems. Im Rahmen der Erfindung ist abweichend allerdings auch möglich, dass eine oder mehrere dieser Datenbanken ganz oder teilweise außerhalb des Systems, beispielsweise in einer Cloud, implementiert sind. Insbesondere kann es sich bei jeder der zwei Datenbanken im weitesten Rahmen der Erfindung grundsätzlich auch um eine öffentlich, z.B. über das Internet zugängliche Datenbank handeln. Jede der zwei Datenbanken kann im Rahmen der Erfindung ihrerseits aus mehreren Unterdatenbanken gebildet sein. Ebenso können die zwei Datenbanken im Rahmen der Erfindung auch in einer gemeinsamen Datenbankstruktur integriert sein.

Das Verfahren und das zugeordnete System sind auf Interaktion mit dem behandelnden Arzt des Patienten ausgerichtet. Bestimmungsgemäß gibt der Arzt die durch eine ausführliche Anamnese bestimmten Patientendaten und das Krankheitsbild als Eingangsgrößen für das Verfahren in das System ein. Des Weiteren stellt der Arzt eine Blutprobe oder Speichelprobe des Patienten für die vorzunehmende Genanalyse zur Verfügung oder gibt die Ergebnisse einer bereits vorhandenen Genanalyse als weitere Eingangsgröße für das Verfahren in das System ein. Der verfahrensgemäß erstellte Bericht wird bestimmungsgemäß durch das System an den Arzt ausgegeben und dient zur Unterstützung des Arztes bei der Planung der Medikation für den Patienten.

Das Verfahren und das zugeordnete System werden vorzugsweise für die Medikationsplanung von multimorbiden Patienten angewendet, die
- gleichzeitig unter Kreislaufstörungen, Diabetes und Depressionen, und/oder
- psychiatrischen Krankheiten (z.B. Depression, Epilepsie, Schizophrenie, Angststörungen, etc.), und/oder
- Krebs leiden.

Die Erfindung ist hierauf aber nicht beschränkt.

Nachfolgend werden Ausführungsbeispiele der Erfindung anhand einer Zeichnung näher erläutert. Darin zeigen:
- Fig. 1: in einem schematischen Blockschaltbild ein System zur Unterstützung der Planung einer mehrere Medikamente umfassenden Medikation für einen Patienten P in einer ersten Ausführungsform, und
- Fig. 2: in Darstellung gemäß Fig. 1 das System in einer zweiten Ausführungsform.

Einander entsprechende Teile und Größen sind in allen Figuren stets mit gleichen Bezugszeichen versehen.

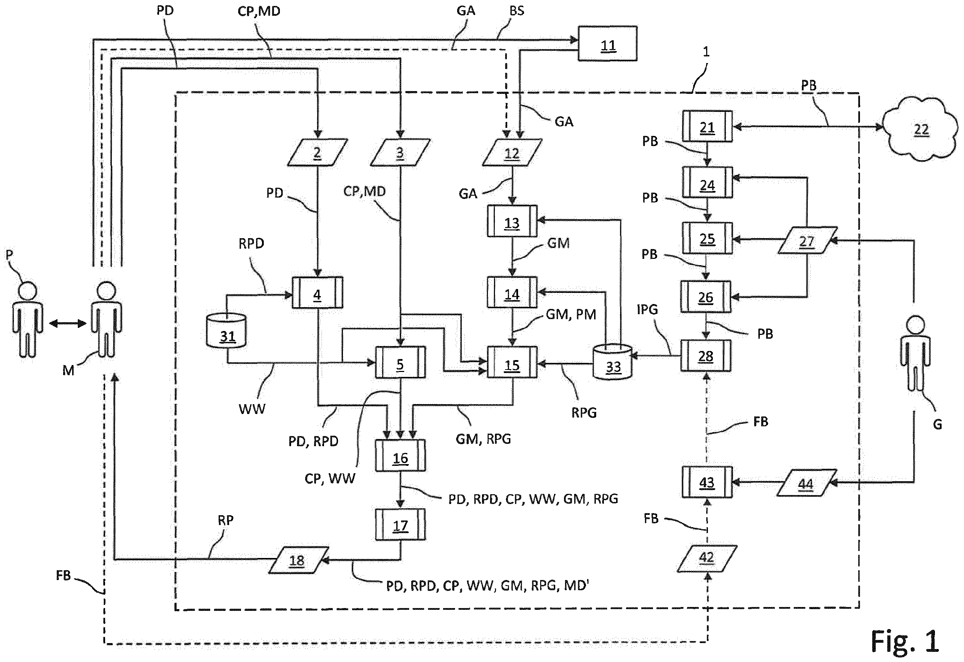

Das System 1 umfasst in dem Ausführungsbeispiel gemäß Fig. 1 ein Eingabemodul 2 für Patientendaten PD eines Patienten P. Die Patientendaten PD enthalten beispielsweise folgende Angaben: Alter, Geschlecht, Körpergewicht, Körpergröße, Blutgruppe, Angaben zum Konsum von Lebensmitteln (Konsum von Obst/Gemüse, Nikotinkonsum, Alkoholkonsum, Koffeinkonsum, Drogenkonsum, etc.), und/oder Lebensumstände (Schwangerschaft, Bewegung/Sport, Stress, Strahlungsbelastung, etc.) des Patienten P.

Das System 1 umfasst weiterhin ein Eingabemodul 3 für Daten zu einem Krankheitsbild CP des Patienten P sowie zu der aktuellen Medikation MD des Patienten P. Das Krankheitsbild CP enthält die diagnostizierten Krankheiten und Beschwerden sowie Angaben zur Organfunktion (z.B. Niere und Leber).

Das System 1 umfasst weiterhin ein Modul 4, das dazu eingerichtet ist, Risiken RPD, die mit den Patientendaten PD korreliert sind, durch Zugriff auf eine Wirkstoff- und Interaktionsdatenbank 31 zu ermitteln. Die Risiken RPD enthalten Angaben zu gesundheitsschädlichen Wirkungen (insbesondere in Hinblick auf eine abnormal gesteigerte oder abgeschwächte Wirkung von Wirkstoffen), deren Auftreten bei dem Patienten P aufgrund der Patientendaten PD, insbesondere aufgrund der von dem Patienten P eingenommenen Lebensmittel und sonstiger Lebensumstände mit erhöhter Wahrscheinlichkeit zu erwarten ist. Die Risiken RPD enthalten zudem Angaben zu Neben- und Wechselwirkungen zwischen verschiedenen eingenommenen Lebensmitteln und/oder Lebensumständen.

Das System 1 umfasst weiterhin ein Modul 5, das dazu eingerichtet ist, durch Zugriff auf die Wirkstoff- und Interaktionsdatenbank 31 Wechselwirkungen WW zwischen den Medikamenten der aktuellen Medikation MD zu ermitteln.

Das System 1 umfasst weiterhin ein Eingabemodul 12 zur standardisierten Erfassung des Resultats einer Genanalyse GA des Patienten P.

Das System 1 umfasst weiterhin ein Modul 13 zur Auswertung der Genanalyse GA. Durch das Modul 13 werden genotypologische Merkmale GM aus der Genanalyse GA extrahiert. Die von dem Modul 13 ausgegebenen genotypologischen Merkmale GM enthalten insbesondere Angaben, ob bestimmte in Hinblick auf ihre klinische Relevanz ausgewählte SNPs (z.B. 128 SNPs) in dem Genotyp des Patienten P enthalten sind. Das Modul 13 ermittelt insbesondere, ob das jeweilige SNP homozygot (also in beiden Exemplaren des jeweils zugeordneten Chromosoms) oder heterozygot (also in lediglich einem der beiden Chromosomen-Exemplare) ausgeprägt ist, oder ob keine Variation für das jeweilige SNP innerhalb eines Gens des Patienten P nachgewiesen wurde (ob also das jeweilige Gen in dem sogenannten "Wildtyp" vorliegt).

Für Information zu der Art der genotypologischen Merkmale GM, auf deren Präsenz die Genanalyse GA zu untersuchen ist, greift das Modul 13 auf eine pharmakogenetische Datenbank 33 zu. Das Modul 13 erhält aus der Datenbank 33 also insbesondere Angaben zu denjenigen SNPs, deren Ausprägung zu überprüfen ist. Diese Information wird durch die Datenbank 33 dynamisch vorgegeben und kann entsprechend auch geändert werden. Insbesondere kann die Gruppe der zu überprüfenden SNPs durch Anpassung der in der Datenbank 33 enthaltenen Information erweitert oder in anderer Weise geändert werden, ohne dass hierfür das Modul 13 selbst angepasst werden müsste. Das Modul 13 berücksichtigt dabei, ob die getesteten Gene als Wildtyp (Normalfall für definierte Population) vorliegen, oder ob gefundene SNPs homozygot oder heterozygot ausgeprägt sind. Es gibt die extrahierten genotypologische Merkmale GM an ein Modul 14 weiter.

Das Modul 14 ist dazu eingerichtet, den genotypologischen Merkmalen GM zugehörige phänotypologische Merkmale PM zuzuordnen. Für jedes Gen, dem mindestens eines der getesteten genotypologischen Merkmale GM zugeordnet ist, wird in Abhängigkeit dieser genotypologischen Merkmale GM ein phänotypologisches Merkmal PM zugewiesen. Bei Enzymen kann das phänotypologische Merkmal PM einer vierstufigen Skala entsprechen: "langsame (poor) Metabolisierung", "leicht eingeschränkte (intermediate) Metabolisierung", "normale (extensive) Metabolisierung", und "beschleunigte (ultra rapid) Metabolisierung". Bei Transportern bzw. Rezeptoren wird die mögliche Veränderung der Transportaktivität bzw. der Rezeptorbindung zugewiesen. Die Information über die Korrelation der genotypologischen Merkmale GM und den daraus resultierenden phänotypologischen Merkmalen PM des zugehörigen Gens und/oder Genkombination bezieht das Modul 14 aus der pharmakogenetische Datenbank 33. Die solchermaßen bestimmten phänotypologischen Merkmale PM werden an ein Modul 15 weitergeleitet.

Die Module 13 und 14 werden übersprungen, wenn die von diesen Modulen 13,14 extrahierte Information in der in das System 1 eingespeisten Genanalyse GA bereits in aufbereiteter Form vorhanden ist.

Das Modul 15 ist zur Ermittlung von pharmakogenetischen Risiken RPG des Patienten P eingerichtet. Die pharmakogenetischen Risiken RPG enthalten Angaben zu Medikamenten aus der aktuellen Medikation MD, für die aufgrund der genetischen Veranlagung des Patienten P (nämlich aufgrund der aus der Genanalyse GA abgeleiteten phänotypologischen Merkmale PM) eine abnormal gesteigerte oder abnormal abgeschwächte Wirkung zu erwarten ist. Dem Modul 15 ist hierzu einerseits die aktuelle Medikation MD zugeführt, woraus das Modul 15 die Information über die von dem Patienten P eingenommenen Medikamente extrahiert. Andererseits greift das Modul 15 auf die Wirkstoff- und Interaktionsdatenbank 31 zu, um die Wirkstoffe dieser Medikamente zu ermitteln. Durch Zugriff auf entsprechende Information aus der pharmakogenetischen Datenbank 33 ermittelt das Modul 15 für jeden dieser Wirkstoffe diejenigen Enzyme, durch die der jeweilige Wirkstoff verarbeitet wird. Zusätzlich oder alternativ werden durch das Modul 15 diejenigen Gene ermittelt, durch die diese Enzyme exprimiert werden. Anhand der phänotypologischen Merkmale PM (nämlich anhand der diesen Enzymen bzw. Genen zugeordneten Aktivitätsgrade) ermittelt das Modul 15 dann die mit der aktuellen Medikation MD verbundenen pharmakogenetischen Risiken RPD.

So erzeugt das Modul 15 im Rahmen der pharmakogenetischen Risiken RPD beispielsweise einen Hinweis auf die Möglichkeit einer abgeschwächten Wirkung eines in der aktuellen Medikation MD enthaltenen Wirkstoffs, wenn dieser Wirkstoff durch ein Enzym metabolisiert wird, das aufgrund einer festgestellten genetischen Variation im Genotyp des Patienten P und der zugeordneten phänotypologischen Merkmale PM abweichend vom Normalfall als "beschleunigte Metabolisierung" ausgewiesen ist.

Dagegen erzeugt das Modul 15 im Rahmen der pharmakogenetischen Risiken RPD beispielsweise einen Hinweis auf die Möglichkeit einer gesteigerten Wirkung eines in der aktuellen Medikation MD enthaltenen Wirkstoffs (und damit der Gefahr einer Überdosierung des jeweiligen Medikaments), wenn dieser Wirkstoff maßgeblich durch ein Enzym metabolisiert wird, das sich aufgrund einer festgestellten genetischen Variation im Genotyp des Patienten P und der zugeordneten phänotypologischen Merkmale PM abweichend vom Normalfall als "langsamer Metabolisierer" oder "leicht eingeschränkter Metabolisierer" erweist.

Ein Modul 16 erstellt automatisch Zusammenfassungen
- der Patientendaten PD,
- der aktuellen Medikation MD,
- des Krankheitsbildes CP,
- der mit den Patientendaten PD korrelierten Risiken RPD,
- der Wechselwirkungen WW der Medikamente aus der aktuellen Medikation MD und
- der pharmakogenetischen Risiken RPG
in einer vorgegeben Datenstruktur.

Optional umfasst das System 1 ein Modul 17 zur Ermittlung möglicher alternativer Medikationen MD'. Das Modul 17 ermittelt hierzu durch (nicht explizit dargestellten) Zugriff auf die Wirkstoff- und Interaktionsdatenbank 31 die therapeutischen Wirkungen der Medikamente der aktuellen Medikation MD. Anhand dieser Wirkungen wählt das Modul 17 aus der Datenbank 31 - sofern vorhanden - mindestens eine Kombination von Medikamenten aus, die sich von der aktuellen Medikation MD unterscheidet, dieser aber hinsichtlich der Gesamtheit der therapeutischen Wirkungen entspricht. Des Weiteren ermittelt das Modul 17 unter Zugriff auf die pharmakogenetische Datenbank 33 und das Modul 15 (nicht dargestellt) auch pharmakogenetische Risiken RPG für die alternative Medikation MD'. Zusätzlich ermittelt das Modul 17 Angaben zur Dosierung und Verabreichung der gegebenenfalls neu ausgewählten Medikamente. Die alternative Medikation MD' kann sich von der aktuellen Medikation MD auch durch eine geänderte Dosierung oder geänderten Einnahmezeiten für bereits aktuell eingenommene Medikamente unterscheiden. Für die alternativen Medikation MD' ermittelt das Modul 17 unter Zugriff auf die Wirkstoff- und Interaktionsdatenbank 31 und das Modul 5 (nicht dargestellt) die zugehörigen Wechselwirkungen WW. Die Ermittlung der alternativen Medikationen MD' wird abgebrochen, wenn sich keine alternative Kombination von Medikamenten, Dosierungen und/oder Einnahmezeiten finden lässt, die hinsichtlich ihrer therapeutischen Wirkungen mit der aktuellen Medikation MD vergleichbar ist. Weiterhin werden in bevorzugter Ausgestaltung des Moduls 17 gefundene alternative Medikationen MD' durch das Modul 17 verworfen, wenn sie mit zu gravierenden Neben- oder Wechselwirkungen WW oder mit zu gravierenden pharmakogenetische Risiken RPG verbunden sind.

Das System 1 umfasst weiterhin ein Ausgabemodul 18, das dazu eingerichtet ist, aus den von dem Modul 16 sowie ggf. dem Modul 17 gesammelten Daten einen Bericht RP in Form eines editierten Textes in natürlicher, menschenlesbarer Sprache zu erzeugen; der Bericht RP enthält dabei folgende Angaben:
- die Patientendaten PD und das Krankheitsbild CP sowie die aktuelle Medikation MD,
- vollständig oder auszugsweise das Ergebnis der Genanalyse GA,
- Interpretation der Genanalyse GA, die daraus abgeleiteten genotypologischen Merkmale GM, die pharmakogenetischen Risiken RPG und die die mit den Patientendaten PD korrelierten Risiken RPD,
- und die Wechselwirkungen WW der Medikamente aus der aktuellen Medikation MD

Optional enthält der Bericht RP zusätzlich folgende weiteren Angaben:
- Erläuterungen zur Interpretation der pharmakogenetischen Risiken RPG, zu pharmakokinetischen Effekten und/oder klinisch relevanten Konsequenzen,
- Hinweise auf einschlägige Referenzliteratur,
- die Angabe der ggf. von dem Modul 17 ermittelten alternativen Medikation mit Angaben zu zugehörigen Wechselwirkungen WW und pharmakogenetischen Risiken RPG (insbesondere Angaben zur Metabolisierung der in der alternativen Medikation MD' enthaltenen Wirkstoffe);

Weiterhin umfasst das System 1 eine Suchmaschine 21 für über das Internet 22 verfügbare und elektronisch durchsuchbare pharmakogenetische Publikationen PB. Die Suchmaschine 1 ist dazu ausgebildet, das Internet 22 automatisch nach vorgegebenen (und nutzerseitig veränderbaren) Kriterien, insbesondere Suchbegriffen, auf einschlägige Publikationen PB auf dem Gebiet der Pharmakogenetik zu durchsuchen. Die Suchmachine 21 wendet vorzugsweise parallel mehrere parallele Suchalgorithmen zu unterschiedlichen Themen (z.B. Genen, Arzneimitteln, etc.) an. Die Suchmaschine 21 erlaubt die Durchführung von zeit- oder termingesteuerten, wiederholten Suchvorgängen ebenso wie die Durchführung einer einfachen Suche nach bestimmten Suchbegriffen.

Ein Modul 24 dient zur Vorsortierung ("Screening") der durch die Suchmaschine 21 gefundenen Publikationen PB hinsichtlich ihrer jeweiligen thematischen Relevanz. Das Modul 24 vergleicht hierzu die zu prüfenden Publikationen PB mit vorgegebenen (und nutzerseitig veränderbaren) Textblöcken. Als Textblock wird hierbei ein komplexer Suchbegriff aus mehreren Suchwörtern bezeichnet, die von dem Modul 24 in verschiedenen logischen Kombinationen (UND-Kombination, ODER-Kombination sowie NICHT-Kombination, d.h. unter Ausschluss einzelner Suchwörter) geprüft werden. Das Modul 24 bewertet jede gefundene Publikation PB danach, wie viele Suchwörter eines Textblocks gefunden wurden. Das Modul 24 speichert dabei ab, wie erfolgreich die verwendeten Textblöcke waren und ist dazu eingerichtet, Vorschläge für neue Textblöcke zu geben. Das Modul 24 optimiert sich somit selbstlernend.

Das System 1 umfasst weiterhin ein Modul 25, in dem thematisch relevante Publikationen PB, in einem ersten Schritt hinsichtlich ihres Evidenzgrades bewertet werden. Der Evidenzgrad der jeweils untersuchten Publikation PB wird dabei vorzugsweise auf die vorstehend beschriebene Weise gemäß einer dreistufigen Skala bewertet als hoch, mittel oder gering. In einem zweiten Schritt wird durch das Modul 25 geprüft, ob die jeweils untersuchte Publikation PB einen pharmakokinetischen Effekt für eine genetische Variation beschreibt, d.h. einen Effekt, der generell die Auswirkung einer genetischen Variation auf die Kinetik eines Medikaments oder Wirkstoffs (z.B. Konzentrationsänderungen des Medikaments oder Wirkstoffs im Blut) betrifft. In einem dritten Schritt wird durch das Modul 25 geprüft, ob in der Publikation PB für die nachgewiesene genetische Variation klinische Konsequenzen (klinische Relevanz) aufgezeigt sind. Klinische Konsequenzen können z.B. im Optimalfall in einer verbesserten Wirkung eines Medikaments bei gleichzeitig geringem Risiko für Neben- und Wechselwirkungen, in einem suboptimalen Fall in einem erhöhten Risiko für Neben- und Wechselwirkungen, oder im Negativfall in einem gesteigerten Risiko für ein Therapieversagen bei gleichzeitig schweren Neben- und Wechselwirkungen bestehen. Eine geprüfte Publikation PB besteht den durch das Modul 25 durchgeführten Test, wenn sie einen nach vorgegebenen Kriterien hinreichenden Evidenzgrad (z.B. mindestens mittleren Evidenzgrad) aufweist und mindestens einen pharmakokinetischen Effekt und/oder klinische Konsequenzen für die nachgewiesene genetische Variation beschreibt.

Durch ein Modul 26 werden Publikationen PB, die das Modul 25 erfolgreich durchlaufen haben, gesammelt und in einem Redundanztest vergleichend auf inhaltliche Übereinstimmung ausgewertet. Sofern eine bestimmte Anzahl von Publikationen PB (insbesondere mindestens zwei Publikationen PB) zu einer übereinstimmenden Thematik, insbesondere bezüglich desselben pharmakogenetischen Zusammenhangs, das Modul 25 erfolgreich durchlaufen haben, wird der Inhalt dieser Publikationen PB zur Aufnahme in die pharmakogenetische Datenbank 33 freigegeben.

Das System 1 umfasst ein Eingabemodul 27, über das ein pharmakogenetischer Experte G (oder ein Expertengremium) die durch die Module 24, 25 und 26 durchgeführten Evaluierungsprozesses steuern, überwachen und anpassen können.

Ein Modul 28 des Systems 1 extrahiert aus den von dem Modulen 24 bis 27 selektierten Publikationen PB die relevante pharmakogenetische Information IPG und trägt diese nach einer automatischen Konsistenzprüfung in einer vorgegebenen Datenstruktur in die pharmakogenetische Datenbank 33 ein.

Optional umfasst das System 1 ferner ein Eingabemodul 42 zur Erfassung von Rückmeldungen FB zum Ergebnis einer in dem Bericht RP vorgeschlagenen alternativen Medikation MD'; sowie ein Modul 43 zur Qualitätskontrolle der über das Eingabemodul 42 erfassten Rückmeldungen FB. Das Modul 43 überprüft die Qualität der erfassten Rückmeldungen FB vorzugsweise automatisch auf Konsistenz und Vollständigkeit. Über ein Eingabemodul 44 kann der pharmakogenetische Experte G bzw. das Expertengremium diesen Kontrollprozess überprüfen und anpassen.

Die Eingabemodule 2,3,12, 26 und 42 sowie das Ausgabemodul 18 sind als GUI, insbesondere als Web-Interface) ausgebildet.

Dem System 1 ist ein Genlabor 11 zur Durchführung der Genanalyse GA des Patienten P, insbesondere mittels Polymerase-Kettenreaktion (Polymerase Chain Reaction, kurz PCR) oder mittels Sequenzierung zugeordnet.

Voraussetzung für die bestimmungsgemäße Durchführung des durch das System 1 durchgeführten Verfahrens ist eine ausführliche Anamnese des Patienten P durch den behandelnden Arzt M. Der Arzt M gibt die Ergebnisse dieser Anamnese in Form der Patientendaten PD, des Krankheitsbildes CP und der aktuellen Medikation MD in das System 1 ein. Er greift hierzu über das Internet auf die Eingabemodule 2 und 3 zu. Weiterhin sendet der Arzt M eine Blutprobe BS des Patienten P an das Genlabor 11, das diese Blutprobe BS der Genanalyse GA unterzieht und die Ergebnisse dieser Genanalyse GA über das Eingabemodul 12 in das System 1 eingibt. Sofern dem Arzt M bereits eine Genanalyse GA des Patienten P vorliegt, kann er diese abweichend auch direkt über das Eingabemodul 12 in das System 1 eingeben. Das Eingabemodul 12 weist hierzu eine konfigurierbare Schnittstelle auf, über die Genanalysen GA verschiedenartiger Sequenziermaschinen in standardisierter Form in das System 1 importierbar sind.

Das Modul 4 extrahiert aus der Wirkstoff- und Interaktionsdatenbank 31 die mit den eingegebenen Patientendaten PD korrelierten Risiken RPD. Das Modul 5 extrahiert aus der Wirkstoff- und Interaktionsdatenbank 31 die Wechselwirkungen WW der Medikamente aus der aktuellen Medikation MD.

Durch das Modul 13 werden in der vorstehend beschriebenen Weise bestimmte genotypologische Merkmale GM aus der Genanalyse GA extrahiert. Das Modul 14 ordnet diesen genotypologischen Merkmalen GM zugehörige phänotypologische Merkmale PM zu. Das Modul 15 ermittelt in Abhängigkeit der aktuellen Medikation MD und der phänotypologischen Merkmale PM sowie unter Zugriff auf Wirkstoff- und Interaktionsdatenbank 31 und die pharmakogenetische Datenbank 33 die pharmakogenetischen Risiken RPG.

Die Patientendaten PD, die sich daraus ergebenden Risiken RPD, das Krankheitsbild CP, die aktuelle Medikation MD und deren Wechselwirkungen WW, die genotypologischen Merkmale GM und die daraus abgeleiteten pharmakogenetischen Risiken RPG werden durch das Modul 16 gesammelt.

Das Modul 17 ermittelt mehrere alternative Medikationen MD', die hinsichtlich ihrer therapeutischen Wirkungen der aktuellen Medikation MD entsprechen, sowie zugeordnete pharmakogenetische Risiken RPG und zugeordnete Wechselwirkungen WW.

Das Ausgabemodul 18 erstellt automatisch aus der durch die Module 16 und 17 gesammelten Information den schriftlichen Bericht RP und gibt diesen Bericht RP, beispielsweise mittels einer automatisch generierten E-Mail, an den Arzt M aus.

Anhand der in dem Bericht RP enthaltenen Information entscheidet der Arzt, ob die aktuelle Medikation MD des Patienten P beibehalten oder geändert werden soll. Insbesondere hat der Arzt M hierbei die Möglichkeit, eine der in dem Bericht RP enthaltenen alternativen Medikationen MD' anzuwenden.

Sofern sich der Arzt M für die Anwendung einer der alternativen Medikationen MD' entscheidet, hat er die Möglichkeit, das Ergebnis der Anwendung dieser alternativen Medikationen MD' auf den Patienten P nach Ablauf eines gewissen Beobachtungszeitraums (z.B. nach 2 bis 4 Wochen) in dem System 1 zu bewerten.

Hierzu gibt der Arzt M über das Eingabemodul 42 eine Rückmeldung FB in das System 1 ein. Die Rückmeldung FB enthält beispielsweise Angaben dazu, ob bezüglich der in dem Krankheitsbild CP angegebenen Krankheiten oder Beschwerden nach der Anwendung der alternativen Medikationen MD' eine Verbesserung oder Verschlechterung eingetreten ist.

Die Rückmeldung FB wird durch das Modul 43 automatisch auf Vollständigkeit und Plausibilität geprüft. Sofern die Rückmeldung FB diese Qualitätsprüfung erfolgreich durchläuft, wird der Inhalt der Rückmeldung FB durch das Modul 43 für die Verwendung zur Aktualisierung der pharmakogenetischen Datenbank 33 freigegeben.

Ggf. wird der Inhalt der Rückmeldung FB durch das Modul 28 extrahiert und in die pharmakogenetische Datenbank 33 eingepflegt.

Die Aktualisierung der pharmakogenetischen Datenbank 33 erfolgt fortlaufend durch die Suchmaschine 21 und die Module 24 und 28 auf die vorstehend beschriebene Weise.

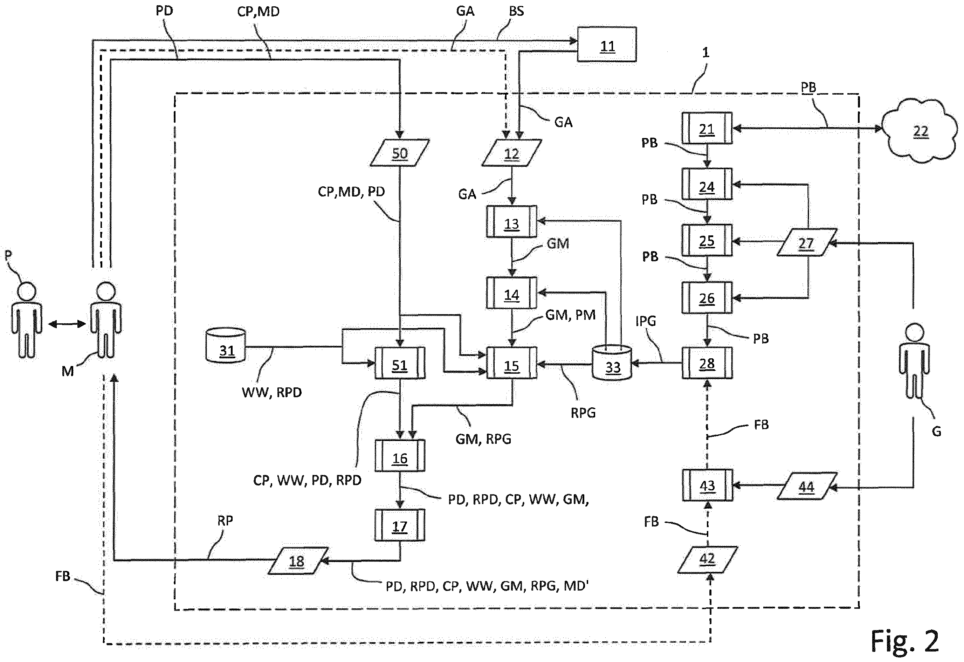

Die in Fig. 2 dargestellte Variante des Systems 1 unterscheidet sich von der vorstehend beschriebenen Ausführungsform des Systems 1 dadurch, dass anstelle der beiden Eingabemodule 2 und 3 ein gemeinsames Eingabemodul 50 für die Patientendaten PD, das Krankheitsbild CP und die aktuelle Medikation MD vorgesehen ist. Des Weiteren ist statt der Module 4 und 5 ein gemeinsames Modul 51 vorgesehen, das durch Zugriff auf die Wirkstoff- und Interaktionsdatenbank 31 sowohl die mit den Patientendaten PD korrelierten Risiken RPD als auch die Wechselwirkungen WW ermittelt.

Das System 1 gemäß Fig. 2 zeichnet sich dabei dadurch aus, dass medizinische Information (nämlich das Krankheitsbild CP und die Medikation MD) einerseits und in den Patientendaten PD enthaltene Information über Lebensmittelkonsum und sonstige Lebensumstände des Patienten P zusammen und gleichberechtigt verarbeitet werden.

Entsprechend werden durch das Modul 15 hier auch pharmakogenetische Risiken RPG aufgrund von Lebensmitteln und/oder sonstigen Lebensumständen ermittelt. Beispielsweise weist das Modul 15 hier im Rahmen der pharmakogenetische Risiken RPG auch ggf. auf eine genetisch bedingte Strahlungsunverträglichkeit des Patienten P hin, sofern aus den Patientendaten PD hervorgeht, dass der Patient P einer erhöhten Strahlungsbelastung ausgesetzt ist.

Im Übrigen entspricht das System 1 gemäß Fig. 2 dem Ausführungsbeispiel nach Fig. 1.

Die Erfindung wird an den vorstehend beschriebenen Ausführungsbeispielen besonders deutlich. Sie ist gleichwohl auf diese Ausführungsbeispiele aber nicht beschränkt. Vielmehr können weitere Ausführungsformen der Erfindung aus den Ansprüchen und der vorstehenden Beschreibung abgeleitet werden.

### Bezugszeichenliste

- 1: System
- 2,3: Eingabemodul
- 4,5: Modul
- 11: Genlabor
- 12: Eingabemodul
- 13-17: Modul
- 18: Ausgabemodul
- 21: Modul
- 22: Internet
- 24-26: Modul
- 27: Eingabemodul
- 28: Modul
- 31: Wirkstoff- und Interaktionsdatenbank
- 33: (pharmakogenetische) Datenbank
- 42: Eingabemodul
- 43: Modul
- 44: Eingabemodul
- 50,51: Modul

- G: (pharmakogenetischer) Experte
- M: Arzt
- P: Patient

- BS: Blutprobe
- CP: Krankheitsbild
- FB: Rückmeldung
- GA: Genanalyse
- GM: (genotypologische) Merkmale
- IPG: (pharmakogenetische) Information
- MD: (aktuelle) Medikation
- MD': (alternative) Medikation
- PB: Publikation
- PD: Patientendaten
- PM: (phänotypologische) Merkmale
- RP: Bericht
- RPD: Risiken
- RPG: Risiken
- WW: Wechselwirkungen

## Patentansprüche

1. Verfahren zur Unterstützung der Planung einer Medikation (MD) für einen Patienten (P), mit folgenden Schritten:
a) Bereitstellen einer Wirkstoff- und Interaktionsdatenbank (31), die
- Informationen zu Wirkstoffen und Anwendungsangaben von Medikamenten, und/oder
- Informationen zu Wirkstoffen von Lebensmitteln, umfassend Nahrungsmittel, Nahrungsergänzungsmittel, Genussmittel, Kräuter und/oder Betäubungsmittel, und/oder
- Informationen zu Auswirkungen von Lebensumständen, umfassend Alter, Geschlecht, Körpergewicht, Schwangerschaft, Organfunktionen und/oder Strahlungsbelastung, auf die Wirkung von Medikamenten bzw. Lebensmitteln,
umfasst,
b) Bereitstellen einer pharmakogenetischen Datenbank (33), die Informationen zu genetischen Einflüssen auf die Metabolisierung und/oder den Transport von Wirkstoffen aus Medikamenten und/oder Lebensmitteln und/oder deren Bindung an Rezeptoren enthält,
c) Erfassen von Patientendaten (PD) eines Patienten (P), die Angaben zur Einnahme bestimmter Lebensmittel und/oder Angaben zu Lebensumständen umfassen, mittels eines hierzu eingerichteten Eingabemoduls (2,50),
d) Erfassen von Angaben zum Krankheitsbild (CP) des Patienten, umfassend Angaben zu diagnostizierten Krankheiten und/oder Beschwerden, sowie das Erfassen einer aktuellen Medikation (MD) des Patienten, mittels eines hierzu eingerichteten Eingabemoduls (3,50),
e) Ermitteln von mit den Patientendaten (PD) korrelierten Risiken (RPD) für eine abnormal verstärkte oder abgeschwächte Wirkung von Wirkstoffen der aktuellen Medikation (MD) und/oder der eingenommenen Lebensmittel aus der Wirkstoff- und Interaktionsdatenbank (31) mittels eines hierzu eingerichteten Moduls (4,51),
f) Erfassen der Ergebnisse einer Genanalyse (GA) des Patienten (P) mittels eines hierzu eingerichteten Eingabemoduls (12),
g) Extrahieren von genotypologischen Merkmalen (GM) aus der Genanalyse (GA) mittels eines hierzu eingerichteten Moduls (13),
h) Ermitteln von mit den genotypologischen Merkmalen (GM) korrelierten pharmakogenetischen Risiken (RPG) für eine abnormal verstärkte oder abgeschwächte Wirkung von Wirkstoffen der aktuellen Medikation (MD) und/oder den eingenommenen Lebensmitteln aus der pharmakogenetischen Datenbank (33) mittels eines hierzu eingerichteten Moduls (15),
i) automatische Ermittlung einer alternativen Medikation (MD') aus der Wirkstoff- und Interaktionsdatenbank (31) anhand der aktuellen Medikation (MD) des Patienten (P) mittels eines hierzu eingerichteten Moduls (17), und
j) automatisches Erstellen eines Berichts (RP) mittels einer Anzahl von hierzu eingerichteten Modulen (16,18) anhand
- der in Schritt e) ermittelten, mit den Patientendaten (PD) korrelierten Risiken (RPD),
- der in Schritt h) ermittelten pharmakogenetischen Risiken (RPG), und
- der in Schritt i) ermittelten alternativen Medikation (MD'),
wobei das Verfahren mittels eines Systems (1) durchgeführt wird, das durch eine auf einer Hardware-Struktur lauffähig implementierte Software gebildet ist, und wobei die das Verfahren durchführenden Module jeweils aus Software und/oder Hardware gebildete Bestandteile des Systems (1) sind, **gekennzeichnet durch**
k) Erfassung einer Rückmeldung (FB) des Patienten (P) zu dem Ergebnis der alternativen Medikation (MD') mittels eines hierzu eingerichteten Eingabemoduls (42) nach Anwendung der alternativen Medikation (MD') auf den Patienten (P), und
l) Aktualisierung der pharmakogenetischen Datenbank (33) anhand der Rückmeldung (FB) mittels eines hierzu eingerichteten Moduls (28).

2. Verfahren nach Anspruch 1,
- wobei die Wirkstoff- und Interaktionsdatenbank (31) zusätzlich Informationen zu Wechselwirkungen (WW) zwischen Medikamenten und/oder Lebensmitteln oder Wechselwirkungen zwischen den jeweils darin enthaltenen Wirkstoffen enthält, und
- wobei Wechselwirkungen (WW) zwischen den in der aktuellen Medikation (MD) enthaltenen Medikamenten und/oder Lebensmitteln bzw. Wirkstoffen aus der Wirkstoff- und Interaktionsdatenbank (31) ermittelt und in dem Bericht (RP) angegeben werden.

3. Verfahren nach Anspruch 2,
- wobei aus der Wirkstoff- und Interaktionsdatenbank (31) Wechselwirkungen (WW) der in der alternativen Medikation (MD') enthaltenen Medikamente oder Wirkstoffe ermittelt werden,
- wobei aus der pharmakogenetischen Datenbank (33) pharmakogenetische Risiken (RPG) für eine abnormal verstärkte oder abgeschwächte Wirkung von in der alternativen Medikation (MD') enthaltenen Wirkstoffen ermittelt werden, und
- wobei in dem Bericht (RP) die alternative Medikation (MD') zusammen mit den zugehörigen Wechselwirkungen (WW) und den zugehörigen pharmakogenetischen Risiken (RPG) angegeben wird.

4. Verfahren nach einem der Ansprüche 1 bis 3,
in dem mittels einer Suchmaschine (21) im Internet (22) pharmakogenetische Publikationen (PB) recherchiert werden, um die pharmakogenetische Datenbank (33) zu aktualisieren.

5. Verfahren nach Anspruch 4,
wobei jede recherchierte Publikation (PB) in einem mehrstufigen Validierungsprozess auf inhaltliche Relevanz, Evidenzgrad, die Beschreibung mindestens eines pharmakokinetischen Effekts und/oder klinischen Konsequenz bezüglich einer genetischen Variation sowie auf inhaltliche Übereinstimmung mit mindestens einer weiteren Publikation (PB) geprüft wird, und wobei der Inhalt der Publikation (PB) nur dann zur Aktualisierung der pharmakogenetische Datenbank (33) herangezogen wird, wenn die Publikation (PB) die Anforderungen aller Stufen des Validierungsprozesses erfüllt.

6. System (1) zur Unterstützung der Planung einer Medikation (MD) für einen Patienten (P),
a) mit einem Eingabemodul (2,50), das dazu eingerichtet ist, Patientendaten (PD), umfassend Angaben zur Einnahme bestimmter Lebensmittel sowie Angaben zu Lebensumständen des Patienten zu erfassen,
b) mit einem Eingabemodul (3,50), das dazu eingerichtet ist, Angaben zum Krankheitsbild (CP) des Patienten (P), umfassend Angaben zu diagnostizierten Krankheiten und/oder Beschwerden, sowie eine aktuelle Medikation (MD) des Patienten (P) zu erfassen,
c) mit einem Eingabemodul (12), das dazu eingerichtet ist, eine Genanalyse (GA) des Patienten (P) zu erfassen,
d) mit einem Modul (4,51), das dazu eingerichtet ist, in Abhängigkeit der erfassten Patientendaten (PD) damit korrelierte Risiken (RPD) für einer abnormal verstärkte oder abgeschwächte Wirkung von Wirkstoffen der aktuellen Medikation (MD) und/oder der eingenommenen Lebensmittel aus einer Wirkstoff- und Interaktionsdatenbank (31) zu ermitteln,
e) mit einem Modul (13), das dazu eingerichtet ist, genotypologische Merkmale (GM) aus der Genanalyse (GA) zu ermitteln,
f) mit einem Modul (15), das dazu eingerichtet ist, mit den genotypologischen Merkmalen (GM) korrelierte pharmakogenetische Risiken (RPG) für eine abnormal verstärkte oder abgeschwächte Wirkung von in der aktuellen Medikation (MD) und/oder den eingenommenen Lebensmitteln enthaltenen Wirkstoffen aus der pharmakogenetischen Datenbank (33) zu ermitteln,
g) mit einem Modul (17), das dazu eingerichtet ist, anhand der aktuellen Medikation (MD) des Patienten (P) aus der Wirkstoff- und Interaktionsdatenbank (31) automatisch mindestens eine alternative Medikation (MD') zu ermitteln, und
h) mit einer Anzahl von Modulen (16,18), die dazu eingerichtet sind, automatisch einen Bericht (RP) anhand
- der in Schritt d) ermittelten, mit den Patientendaten (PD) korrelierten Risiken (RPD),
- der in Schritt f) ermittelten pharmakogenetischen Risiken (RPG) und
- der in Schritt g) ermittelten die alternative Medikation (MD') zu erstellen,
wobei das System (1) durch eine auf einer geeigneten Hardware-Struktur lauffähig implementierte Software gebildet ist, und wobei die Module jeweils aus Software und/oder Hardware gebildete Bestandteile des Systems (1) sind, **gekennzeichnet durch**
i) ein Eingabemodul (42), das dazu eingerichtet ist, nach Anwendung der alternativen Medikation (MD') auf den Patienten (P) eine Rückmeldung (FB) zu dem Ergebnis der alternativen Medikation (MD') zu erfassen, und
j) ein Modul (28) zur Aktualisierung der pharmakogenetischen Datenbank (33) anhand der Rückmeldung (FB).

7. System (1) nach Anspruch 6,
mit einem Modul (5,51), das dazu eingerichtet ist, Wechselwirkungen (WW) zwischen den in der aktuellen Medikation (MD) enthaltenen Medikamenten und/oder den von dem Patienten (P) eingenommenen Lebensmitteln oder zwischen den darin jeweils enthaltenen Wirkstoffen zu ermitteln, und wobei die den Bericht (RP) erstellenden Module (16,18) dazu eingerichtet sind, die ermittelten Wechselwirkungen (WW) in dem Bericht (RP) anzugeben.

8. System (1) nach Anspruch 7,
wobei das Modul (17) zur Ermittlung der alternativen Medikation (MD') im Einzelnen dazu eingerichtet ist,
- aus der Wirkstoff- und Interaktionsdatenbank (31) Wechselwirkungen (WW) der in der alternativen Medikation (MD') enthaltenen Medikamente zu ermitteln,
- aus der pharmakogenetischen Datenbank (33) pharmakogenetische Risiken (RPG) für eine abnormal verstärkte oder abgeschwächte Wirkung von in der alternativen Medikation (MD') enthaltenen Wirkstoffen zu ermitteln, und
und wobei die den Bericht (RP) erstellenden Module (16,18) dazu eingerichtet sind, die alternative Medikation (MD') zusammen mit den zugehörigen Wechselwirkungen (WW) und der zugehörigen pharmakogenetischen Risiken (RPG) in dem Bericht (RP) anzugeben.

9. System (1) nach einem der Ansprüche 6 bis 8,
mit einer Suchmaschine (21), die dazu eingerichtet ist, im Internet (22) pharmakogenetische Publikationen (PB) zu recherchieren, und mit einem Modul (28), das dazu eingerichtet ist, die pharmakogenetische Datenbank (33) mit Inhalten der recherchierten Publikationen (PB) zu aktualisieren.

10. System (1) nach Anspruch 9,
mit einer Anzahl von Modulen (24-27), die dazu eingerichtet sind, die recherchierten Publikationen (PB) in einem mehrstufigen Validierungsprozess auf
- inhaltliche Relevanz,
- Evidenzgrad,
- die Beschreibung mindestens eines pharmakokinetischen Effekts und/oder einer klinischen Konsequenz bezüglich einer genetischen Variation sowie
- auf inhaltliche Übereinstimmung mit mindestens einer weiteren Publikation (PB)
zu prüfen, und den Inhalt der jeweils geprüften Publikation (PB) nur dann zur Aktualisierung der pharmakogenetische Datenbank (33) freizugeben, wenn die Publikation (PB) die Anforderungen aller Stufen des Validierungsprozesses erfüllt.

## Claims

1. Procedure to support medication (MD) planning for a patient (P), with the following steps:
a) Providing an active substance and interaction database (31) that includes
- information on active substances and indications for use of medications, and/or
- information on active substances of foods, comprising food products, dietary supplements, stimulants, herbs and/or narcotics, and/or
- information on the effects of living conditions, including age, gender, body weight, pregnancy, organ function, and/or radiation exposure, on the effects of drugs or foods,
b) providing a pharmacogenetic database (33) containing information on genetic influences on the metabolization and/or transport of active substances from drugs and/or food and/or their binding to receptors,
c) recording patient data (PD) of a patient (P), which include information on the intake of certain foods and/or information on living conditions, by means of an input module (2,50) arranged for this purpose,
d) recording information on the patient's clinical picture (CP), including details of diagnosed illnesses and/or complaints, as well as recording the patient's current medication (MD) by means an input module (3,50) arranged for this purpose,
e) determining risks (RPD) correlated with patient data (PD) for an abnormally enhanced or attenuated effect of active substances of the current medication (MD) and/or the consumed foods from the active substance and interaction database (31) by means of a module (4,51) arranged for this purpose,
f) recording the results of a genetic analysis (GA) of the patient (P) by means of an input module (12) arranged for this purpose,
g) extracting genotypological features (GM) from the genetic analysis (GA) by means of a module (13) arranged for this purpose,
h) determining pharmacogenetic risks (RPG) correlated with the genotypological characteristics (GM) for an abnormally enhanced or attenuated effect of active substances of the current medication (MD) and/or the consumed foods from the pharmacogenetic database (33) by means of a module (15) arranged for this purpose,
i) automatically determining an alternative medication (MD') from the active substance and interaction database (31) on the basis of the current medication (MD) of the patient (P) by means of a module (17) arranged for this purpose, and
j) automatic generation of a report (RP) by means of a number of modules (16, 18) arranged for this purpose on the basis of
- the patient data (PD) correlated risks (RPD) determined in step e),
- the pharmacogenetic risks (RPG) determined in step (h), and
- the alternative medication (MD') determined in step i),
wherein the method is carried out by means of a system (1), which is formed by software implemented in an executable manner on a hardware structure, and wherein the modules carrying out the method are each components of the system (1) formed from software and/or hardware, **characterized by**
k) recording feedback (FB) from the patient (P) on the result of the alternative medication (MD') after application of the alternative medication (MD') to the patient (P) by means of an input module (42) arranged for this purpose, and
l) updating the pharmacogenetic database (33) based on the feedback (FB) by means of a module (28) arranged for this purpose.

2. Method according to claim 1,
- wherein the active substance and interaction database (31) additionally contains information on interactions (WW) between medicaments and/or foods or interactions between the respective active substances contained therein, and
- wherein interactions (WW) between the drugs and/or foods or active substances included in the current medication (MD) are determined from the active substance and interaction database (31) and indicated in the report (RP).

3. Method according to claim 2,
- wherein interactions (WW) of the drugs or active substances included in the alternative medication (MD') are determined from the drug and interaction database (31),
- wherein, from the pharmacogenetic database (33), pharmacogenetic risks (RPG) for abnormally enhanced or attenuated effects of active substances included in the alternative medication (MD') are determined; and
- wherein the report (RP) indicates the alternative medication (MD') along with the associated interactions (WW) and associated pharmacogenetic risks (RPG).

4. Method according to one of claims 1 to 3,
wherein pharmacogenetic publications (PB) on the Internet (22) are researched by means of a search engine (21) to update the pharmacogenetic database (33).

5. Method according to claim 4,
wherein each publication (PB) researched is checked in a multi-stage validation process for content relevance, evidence level, description of at least one pharmacokinetic effect and/or clinical consequence with respect to a genetic variation, and for consistency of content with at least one further publication (PB), and wherein the content of the publication (PB) is used to update the pharmacogenetic database (33) only if the publication (PB) fulfills the requirements of all stages of the validation process.

6. System (1) to support the planning of a medication (MD) for a patient (P),
a) with an input module (2,50) arranged to record patient data (PD), comprising information on the intake of certain foods as well as information on living conditions of the patient,
b) with an input module (3,50) arranged to record information on the clinical picture (CP) of the patient (P), comprising information on diagnosed diseases and/or complaints, as well as a current medication (MD) of the patient (P),
c) with an input module (12) arranged to record a genetic analysis (GA) of the patient (P),
d) with a module (4,51) arranged to determine, as a function of the recorded patient data (PD), risks (RPD) correlated therewith for an abnormally enhanced or attenuated effect of active substances of the current medication (MD) and/or the consumed foods from an active substance and interaction database (31),
e) with a module (13) arranged to determine genotypological characteristics (GM) from the genetic analysis (GA),
f) with a module (15) arranged to determine pharmacogenetic risks (RPG) correlated with the genotypological characteristics (GM) for an abnormally enhanced or attenuated effect of active substances contained in the current medication (MD) and/or the consumed foods from the pharmacogenetic database (33),
g) with a module (17) arranged to automatically determine at least one alternative medication (MD') from the active substance and interaction database (31) on the basis of the current medication (MD) of the patient (P), and
h) with a number of modules (16,18) arranged to automatically generate a report (RP) based on
- the patient data (PD) correlated risks (RPD) determined in step d),
- the pharmacogenetic risks (RPG) determined in step f), and
- the alternative medication (MD') determined in step g).
wherein the system (1) is formed by software implemented in an executable manner on a suitable hardware structure, and wherein the modules are each components of the system (1) formed from software and/or hardware, **characterized by**
i) an input module (42) arranged to record feedback (FB) on the result of the alternative medication (MD') after application of the alternative medication (MD') to the patient (P), and
j) a module (28) for updating the pharmacogenetic database (33) based on the feedback (FB).

7. System (1) according to claim 6,
with a module (5, 51) arranged to determine interactions (WW) between the drugs contained in the current medication (MD) and/or the foods consumed by the patient (P) or between the active substances contained therein in each case, and wherein the modules (16, 18) generating the report (RP) are arranged to indicate the determined interactions (WW) in the report (RP).

8. System (1) according to claim 7,
wherein the alternative medication (MD') determination module (17) is set up in detail to,
- determine interactions (WW) of the active substances included in the alternative medication (MD') from the active substance and interaction database (31),
- determine pharmacogenetic risks (RPGs) for abnormally enhanced or attenuated effects of active substances included in the alternative medication (MD') from the pharmacogenetic database (33); and
and wherein the report (RP) generating modules (16,18) are arranged to indicate the alternative medication (MD') along with the associated interactions (WW) and the associated pharmacogenetic risk (RPG) in the report (RP).

9. System (1) according to one of claims 6 to 8,
with a search engine (21) arranged to research pharmacogenetic publications (PB) on the Internet (22), and with a module (28) arranged to update the pharmacogenetic database (33) with contents of the researched publications (PB).

10. System according to claim 9,
with a number of modules (24-27) arranged to validate the researched publications (PB) in a multi-stage validation process for
- content relevance,
- evidence level,
- the description of at least one pharmacokinetic effect and/or clinical consequence with respect to a genetic variation, and
- for consistency of content with at least one other publication (PB) and to release the content of each publication (PB) reviewed for updating the pharmacogenetic database (33) only if the publication (PB) fulfills the requirements of all stages of the validation process.

## Revendications

1. Procédé d'aide à la planification d'une médication (MD) pour un patient (P), avec les étapes suivantes :
a) mettre à disposition une base de données de substances actives et d'interactions (31), qui comprend
- des informations sur les substances actives et les indications d'utilisation des médicaments, et/ou
- des informations sur les substances actives de produits alimentaires, comprenant des aliments, des compléments alimentaires, des stimulants, des herbes et/ou des stupéfiants, et/ou
- des informations sur les effets des conditions de vie, y compris l'âge, le sexe, le poids corporel, la grossesse, les fonctions organiques et/ou l'exposition aux rayonnements, sur l'effet des médicaments ou des aliments,
b) mettre à disposition une base de données pharmacogénétique (33) contenant des informations sur les influences génétiques sur la métabolisation et/ou le transport de substances actives provenant de médicaments et/ou d'alimentaires et/ou sur leur liaison à des récepteurs,
c) enregistrer des données de patient (PD) d'un patient (P), qui comprennent des indications sur la prise de certains aliments et/ou des indications sur les conditions de vie, au moyen d'un module de saisie (2,50) prévu à cet effet,
d) enregistrer des informations sur le tableau clinique (CP) du patient, comprenant des informations sur des maladies diagnostiqués et/ou des troubles, et enregistrer une médication actuelle (MD) du patient, au moyen d'un module de saisie (3,50) prévu à cet effet,
e) déterminer des risques (RPD) corrélés aux données de patient (PD) pour un effet anormalement renforcé ou atténué de substances actives de la médication actuelle (MD) et/ou des aliments ingérés à partir de la base de données de substances actives et d'interactions (31) au moyen d'un module (4, 51) prévu à cet effet,
f) enregistrer des résultats d'une analyse génétique (AG) du patient (P) au moyen d'un module de saisie (12) prévu à cet effet,
g) extraire des caractéristiques génotypologiques (GM) de l'analyse génétique (AG) au moyen d'un module (13) prévu à cet effet,
h) déterminer des risques pharmacogénétiques (RPG) corrélés aux caractéristiques génotypologiques (GM) pour un effet anormalement renforcé ou atténué de substances actives de la médication actuelle (MD) et/ou des aliments ingérés à partir de la base de données pharmacogénétiques (33) au moyen d'un module (15) prévu à cet effet,
i) déterminer automatiquement une médication alternative (MD') à partir de la base de données de substances actives et d'interactions (31) sur la base de la médication actuelle (MD) du patient (P) au moyen d'un module (17) prévu à cet effet, et
j) générer automatiquement d'un rapport (RP) au moyen d'un certain nombre de modules (16, 18) prévus à cet effet, sur la base
- des risques (RPD) corrélés aux données du patient (DP) déterminés à l'étape e),
- des risques pharmacogénétiques (RPG) déterminés à l'étape h), et
- de la médication alternative (MD') déterminée à l'étape i),
le procédé étant mis en oeuvre au moyen d'un système (1), qui est formé par un logiciel implémenté de manière à pouvoir fonctionner sur une structure matérielle, et les modules exécutant le procédé étant des composants du système (1) formés respectivement par un logiciel et/ou un matériel, **caractérisé par**
k) enregistrer une réponse (FB) du patient (P) au résultat de la médication alternative (MD') au moyen d'un module de saisie (42) prévu à cet effet après application de la médication alternative (MD') au patient (P), et
l) mettre à jour de la base de données pharmacogénétique (33) sur la base de la réponse (FB) au moyen d'un module (28) prévu à cet effet.

2. Procédé selon la revendication 1,
- dans lequel la base de données de substances actives et d'interactions (31) contient en outre des informations sur des interactions (WW) entre des médicaments et/ou des aliments ou des interactions entre les substances actives, qui y sont respectivement contenues, et
- dans lequel les interactions (WW) entre les médicaments et/ou les aliments ou les substances actives contenus dans la médication actuelle (MD) sont déterminées à partir de la base de données des substances actives et des interactions (31) et indiquées dans le rapport (RP).

3. Procédé selon la revendication 2,
- dans lequel des interactions (WW) des médicaments ou des substances actives contenus dans la médication alternative (MD') sont déterminées à partir de la base de données des substances actives et des interactions (31),
- dans lequel à partir de la base de données pharmacogénétiques (33) des risques pharmacogénétiques (RPG) pour un effet anormalement renforcé ou atténué de substances actives contenues dans la médication alternative (MD') sont déterminés, et
- dans lequel le rapport (RP) indique la médication alternative (MD') ainsi que les interactions (WW) et les risques pharmacogénétiques (RPG) associés.

4. Procédé selon l'une des revendications 1 à 3,
dans lequel des publications pharmacogénétiques (PB) sont récherchées au moyen d'un moteur de recherche (21) sur Internet (22), afin de mettre à jour la base de données pharmacogénétiques (33).

5. Procédé selon la revendication 4,
dans lequel chaque publication (PB) recherchée est examinée dans un processus de validation à plusieurs niveaux pour la pertinence du contenu, le degré d'évidence, la description d'au moins un effet pharmacocinétique et/ou une conséquence clinique concernant une variation génétique, ainsi que pour la concordance du contenu avec au moins une autre publication (PB), et dans lequel le contenu de la publication (PB) n'est utilisé pour la mise à jour de la base de données pharmacogénétique (33) que si la publication (PB) satisfait aux exigences de toutes les étapes du processus de validation.

6. Système (1) d'aide à la planification d'une médication (MD) pour un patient (P),
a) avec un module de saisie (2, 50), qui est conçu pour saisir des données de patient (PD), comprenant des indications sur la prise de certains aliments ainsi que des indications sur les conditions de vie du patient,
b) avec un module de saisie (3, 50), qui est conçu pour saisir des indications sur le tableau clinique (CP) du patient (P), comprenant des indications sur des maladies diagnostiqués et/ou des troubles, ainsi qu'une médication actuelle (MD) du patient (P),
c) avec un module de saisie (12), qui est conçu pour saisir une analyse génétique (GA) du patient (P),
d) avec un module (4, 51), qui est conçu pour déterminer, en fonction des données de patient (PD) saisies, des risques (RPD) corrélés à celles-ci pour un effet anormalement renforcé ou affaibli de substances actives de la médication actuelle (MD) et/ou des aliments ingérés à partir d'une base de données de substances actives et d'interactions (31),
e) avec un module (13), qui est conçu pour déterminer des caractéristiques génotypologiques (GM) à partir de l'analyse génétique (GA),
f) avec un module (15), qui est conçu pour déterminer, à partir de la base de données pharmacogénétique (33), des risques pharmacogénétiques (RPG) corrélés aux caractéristiques génotypologiques (GM) pour un effet anormalement renforcé ou atténué de substances actives contenues dans la médication actuelle (MD) et/ou les aliments ingérés,
g) avec un module (17), qui est conçu pour déterminer automatiquement au moins une médication alternative (MD') à la base de la médication actuelle (MD) du patient (P) à partir de la base de données de substances actives et d'interactions (31), et
h) avec un certain nombre de modules (16, 18), qui sont conçus pour générer automatiquement un rapport (RP) sur la base
- des risques (RPD) corrélés aux données du patient (DP) déterminés à l'étape d),
- des risques pharmacogénétiques (RPG) déterminés à l'étape f), et
- la médication alternative (MD') déterminée à l'étape g),
dans lequel le système (1) est formé par un logiciel implémenté de manière à pouvoir fonctionner sur une structure matérielle appropriée, et les modules sont des composants du système (1) formés chacun par un logiciel et/ou un matériel. **caractérisé par**
i) un module de saisie (42), qui est adapté pour saisir une réponse (FB) sur le résultat de la médication alternative (MD') après application de la médication alternative (MD') au patient (P), et
j) un module (28) de mise à jour de la base de données pharmacogénétique (33) à partir de la réponse (FB).

7. Système (1) selon la revendication 6,
avec un module (5, 51), qui est conçu pour déterminer des interactions (WW) entre les médicaments contenus dans la médication actuelle (MD) et/ou les aliments ingérés par le patient (P) ou entre les substances actives, qui y sont respectivement contenues, et dans lequel les modules (16, 18) générant le rapport (RP) sont conçus pour indiquer les interactions (WW) déterminées dans le rapport (RP).

8. Système (1) selon la revendication 7,
dans lequel le module (17) pour la détermination de la médication alternative (MD') est conçu pour,
- déterminer les interactions (WW) des médicaments contenus dans la médication alternative (MD') à partir de la base de données de substances actives et d'interactions (31),
- identifier les risques pharmacogénétiques (RPG) d'un effet anormalement accru ou atténué des substances actives contenues dans la médication alternative (MD') à partir de la base de données pharmacogénétiques (33), et
et dans lequel les modules (16, 18) générant le rapport (RP) sont conçus pour indiquer dans le rapport (RP) la médication alternative (MD') ainsi que les interactions (WW) et les risques pharmacogénétiques (RPG) associés.

9. Système (1) selon l'une des revendications 6 à 8,
avec un moteur de recherche (21), qui est conçu pour rechercher sur Internet (22) des publications pharmacogénétiques (PB), et avec un module (28), qui est conçu pour mettre à jour la base de données pharmacogénétique (33) avec les contenus des publications (PB) recherchées.

10. Système selon la revendication 9,
avec un certain nombre de modules (24-27), qui sont conçus pour valider les publications recherchées (PB) dans un processus de validation en plusieurs étapes sur
- la pertinence du contenu,
- le degré d'évidence,
- la description d'au moins un effet pharmacocinétique et/ou d'une conséquence clinique concernant une variation génétique, et
- sur la concordance du contenu avec au moins une autre publication (PB) et de n'autoriser la mise à jour de la base de données pharmacogénétique (33) du contenu de la publication (PB) examinée que si la publication (PB) satisfait aux exigences de toutes les étapes du processus de validation.
